# EUROPEAN PATENT SPECIFICATION

(11) **EP 2 246 797 B1**
(45) Date of publication and mention of the grant of the patent: **15.04.2020**
(21) Application number: 09706391.1
(22) Date of filing: 30.01.2009
(51) Int. Cl.: G16H 40/63

(54) **PERSONALIZED BED, AND A METHOD FOR IMPLEMENTING THE SAME**
PERSONALISIERTES BETT UND VERFAHREN ZU SEINER IMPLEMENTIERUNG
LIT PERSONNALISÉ , ET SON PROCÉDÉ DE MISE EN OEUVRE

(30) Priority: 01.02.2008 KR 20080010820; 30.04.2008 KR 20080040824
(43) Date of publication of application: 03.11.2010
(73) Proprietor: Samsung Electronics Co., Ltd., Suwon-si, Gyeonggi-do, 443-742 (KR)
(72) Inventor: JANG, Woo-Young, Yongin-si Gyeonggi-do 446-712 (KR); LIM, Hyung-Kyu, Yongin-si Gyeonggi-do 446-712 (KR); KIM, Dong-Wook, Yongin-si Gyeonggi-do 446-712 (KR)
(74) Representative: Grünecker Patent- und Rechtsanwälte PartG mbB
(86) International application number: PCT/KR2009/000449
(87) International publication number: WO 2009/096727

(56) References cited:
- WO-A1-2007/102709
- US-A1- 2004 111 045
- US-A1- 2005 143 617
- US-A1- 2005 143 617
- US-A1- 2006 058 587
- US-A1- 2006 183 980
- US-A1- 2007 083 079
- US-A1- 2007 083 079
- US-B2- 7 304 580

## Description

### TECHNICAL FIELD

One or more embodiments of the present invention relates to an apparatus for providing various functions for physical and mental relaxation and for the health care of a user, and a method of implementing the apparatus.

### BACKGROUND ART

Currently, as a side effect of rapid industrial development, the air we breathe contains materials which are harmful to the human body and diseases continuously increase due to physical and mental stress. Also, electromagnetic waves generated by various electronic devices greatly threaten people's health. Accordingly, electronic appliances, such as washing machines releasing anions, are developed and introduced in consideration of people's health, instead of improving their performance.

Document US 2007/083079 A1 is directed at an apparatus and/or a method for inducing sound sleep and waking. A protocol is selected to control a sound sleep and waking environment depending on a sleep type selected by a user. The sleep environment of the user is adjusted according to the selected protocol.

Document US 2005/143617 A1 describes a sleep and environment control method and a system. The sleep systems are designed to gather sleep and body data from individuals as they are sleeping as well as environmental data surrounding the sleep systems.

Document WO 2007/102709 A1 is directed to a portable biofeedback exercise prescription apparatus and a biofeedback exercise prescription method using the same.

### SUMMARY OF THE INVENTION

The invention is described in the claims. The embodiments which do not fall within the scope of the claims are to be interpreted as examples useful for understanding the invention. The invention provides a method of implementing a personally customized bed according to claim 1. The invention further provides a personally customized bed according to claim 12. Preferred embodiments are defined in the dependent claims.

### DESCRIPTION OF THE DRAWINGS

FIG. 1 is a diagram illustrating a personally customized bed and an installation environment of the personally customized bed, according to an embodiment of the present invention.
FIG. 2 is a structural view of the personally customized bed illustrated in FIG. 1, according to an embodiment of the present invention.
FIG. 3 is a diagram showing an example of a remote medical service provided through a communications unit illustrated in FIG. 2, according to an embodiment of the present invention.
FIG. 4 is a flowchart of a method of implementing a personally customized bed, according to an embodiment of the present invention.
FIG. 5 is a flowchart of a method of managing a sound sleep of a user on the personally customized bed illustrated in FIG. 2, as an example of possible scenarios in terms of control functions illustrated in FIG. 4, according to an embodiment of the present invention.

### DETAILED DESCRIPTION OF THE INVENTION

Hereinafter, the present invention will be described in detail by explaining embodiments of the invention with reference to the attached drawings.

In general, electronic appliances such as televisions (TVs), refrigerators, and washing machines are home electronic devices for providing convenience or joy to users. On the other hand, furniture, such as beds, chairs, and sofas, is used to support the human body. In the present invention, "electronic furniture" means furniture for providing various electronic functions, namely, complex objects in which electronic constructions of electronic appliances are combined with conventional furniture. In the following description of the present invention, an electronic bed will be described as an example of the electronic furniture. However, it will be understood by those of ordinary skill in the art that the present invention may be applied to any other type of furniture, such as a chair or a sofa.

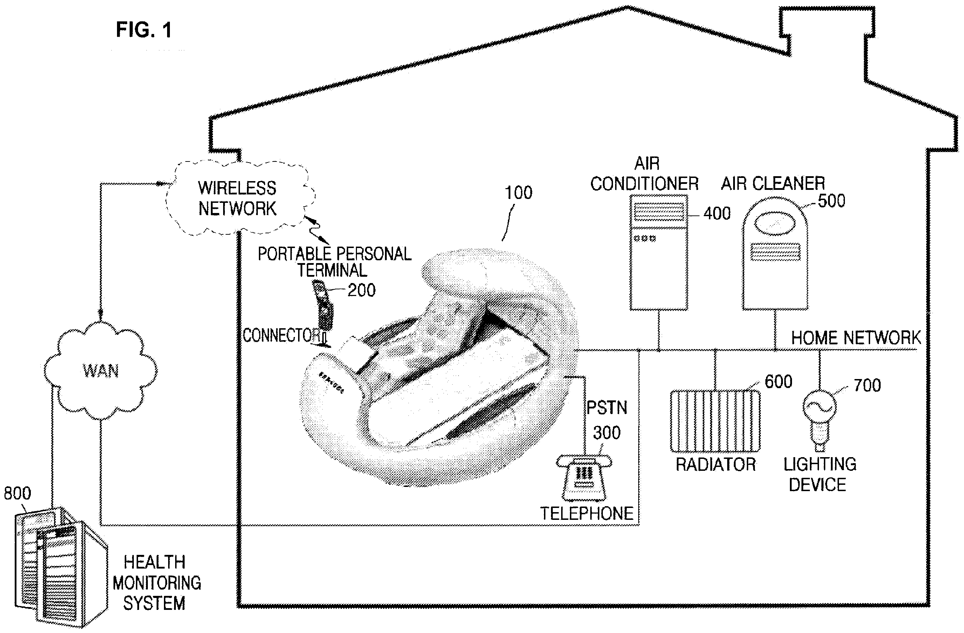

FIG. 1 is a diagram illustrating a personally customized bed 100 and an installation environment of the personally customized bed 100, according to an embodiment of the present invention.

Referring to FIG. 1, the personally customized bed 100 includes a connector on which a portable personal terminal 200 may be mounted, and is connected to a home network. Thus, the personally customized bed 100 may be connected to a health monitoring system 800 located at a node on an external wide area network (WAN), by using a wireless communication function of the portable personal terminal 200 or by using a wired communication function of the home network. Also, the personally customized bed 100 may be connected to an air conditioner 400, an air cleaner 500, a radiator 600, or a lighting device 700, through the home network. Furthermore, the personally customized bed 100 may be connected to a public switched telephone network (PSTN) through a telephone 300 that is connected to the personally customized bed 100.

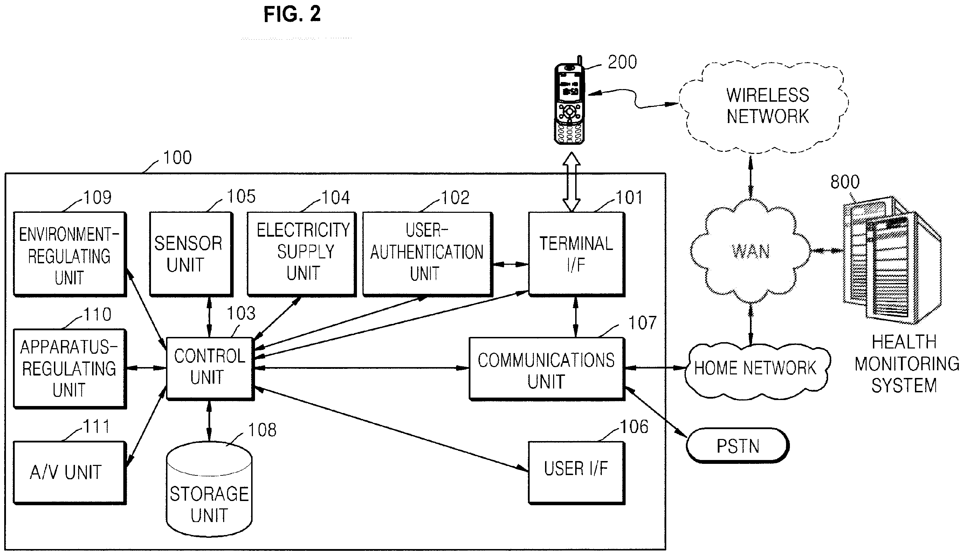

FIG. 2 is a structural view of the personally customized bed 100 illustrated in FIG. 1, according to an embodiment of the present invention. FIG. 2 will be described in conjunction with FIG. 1.

Referring to FIG. 2, the personally customized bed 100 includes a terminal interface (I/F) 101, a user-authentication unit 102, a control unit 103, an electricity supply unit 104, a sensor unit 105, a user I/F 106, a communications unit 107, a storage unit 108, an environment-regulating unit 109, an apparatus-regulating unit 110, and an audio/video (A/V) unit 111. The personally customized bed 100 to be described below is just an example of personally customized electronic furniture and as already mentioned above, the personally customized electronic furniture may include any other type of furniture similar to the personally customized bed 100, such as a personally customized chair and a personally customized sofa.

The terminal I/F 101 detects whether the portable personal terminal 200 is connected to the personally customized bed 100. In particular, a connector into which the portable personal terminal 200 is inserted, is mounted on the terminal I/F 101. That is, by inserting the portable personal terminal 200 into the connector, the portable personal terminal 200 is connected to the personally customized bed 100. Here, representative examples of the portable personal terminal 200 are a cellular phone having a communication function, a personal digital assistant (PDA), an MPEG audio layer 3 (MP3) player, a notebook computer, and a palm top computer.

Also, according to the current embodiment of the present invention, the portable personal terminal 200 may have a function of measuring biological signals from the body of a user. For example, in the current embodiment of the present invention, the portable personal terminal 200 may be a portable medical device having a communication function. In general, the portable medical device means a medical device which is worn on a wrist, similar to a wristwatch for example, and which measures biological signals such as blood sugar level, blood pressure, and electrocardiogram (ECG). In the current embodiment of the present invention, a communication function is added to the portable medical device. Furthermore, those of ordinary skill in the art may understand that any other type of terminals similar to the portable medical device may be used.

The user-authentication unit 102 determines whether a user of the portable personal terminal 200 is authorized to use the personally customized bed 100, by checking a personal identification code of the portable personal terminal 200 that is connected to the personally customized bed 100. The personally customized bed 100 according to the current embodiment of the present invention may be located in a private house or in a public place such as a hospital. If the personally customized bed 100 is located in a public place, the user-authentication unit 102 is necessary for preventing indiscriminate use of the personally customized bed 100 so that only a user who is authorized to use the personally customized bed 100 by, for example, paying a fee for the personally customized bed 100, may use the personally customized bed 100. However, if the personally customized bed 100 is located in a private house, the user-authentication unit 102 may not be necessary.

If the terminal I/F 101 detects that the portable personal terminal 200 is not connected to the personally customized bed 100, or if the user-authentication unit 102 determines that the user of the portable personal terminal 200 is not authorized to use the personally customized bed 100, the control unit 103 controls a power supply function of the electricity supply unit 104 to be maintained in a stand-by mode for providing power only to the terminal I/F 101, the user-authentication unit 102, and the control unit 103. If the terminal I/F 101 detects that the portable personal terminal 200 is connected to the personally customized bed 100, the control unit 103 controls the power supply function of the electricity supply unit 104 to be changed from the stand-by mode to an active mode for providing power not only to the terminal I/F 101, the user-authentication unit 102, and the control unit 103 but also to other elements of the personally customized bed 100. Alternatively, the control unit 103 may control the power supply function of the electricity supply unit 104 from the stand-by mode to the active mode in accordance with whether the user is seated or is lying on the personally customized bed 100, that is, according to a pressed state of air cells in a mattress of the personally customized bed 100, instead of detecting whether the portable personal terminal 200 is connected to the personally customized bed 100. In this case, user authentication may be performed by using voice recognition technology.

When the personally customized bed 100 is located in a public place, the control unit 103 controls the power supply function of the electricity supply unit 104 to be changed from the stand-by mode to the active mode only if the user-authentication unit 102 determines that the user of the portable personal terminal 200 is authorized to use the personally customized bed 100. Also, in the active mode, if the terminal I/F 101 detects that the portable personal terminal 200 is not connected to the personally customized bed 100, the control unit 103 controls the power supply function of the electricity supply unit 104 to be changed from the active mode to the stand-by mode. That is, the personally customized bed 100 starts the active mode for operating all functions if the user inserts the portable personal terminal 200 into the connector of the terminal I/F 101, and the personally customized bed 100 starts the stand-by mode only for detecting whether the portable personal terminal 200 is connected to the personally customized bed 100 if the user separates the portable personal terminal 200 from the connector of the terminal I/F 101.

Also, the control unit 103 controls a communication function of the communications unit 107 to download personal data of the user from the portable personal terminal 200, to connect the personally customized bed 100 to the health monitoring system 800 located in a remote place, through a network, or to transmit a certain message to a rescue party (for example, 911) or to a physician in charge by phone through a public switched telephone network (PSTN). Furthermore, the control unit 103 controls a sensing function of the sensor unit 105 to sense an environment around the personally customized bed 100 or to measure biological signals of the user. As such, the control unit 103 controls one or more functions provided by the personally customized bed 100 based on at least one of the personal data downloaded by the communications unit 107, remote control information received by the communications unit 107, environment information obtained by the sensor unit 105, and direct control information received by the user I/F 106. According to the current embodiment of the present invention, the functions provided by the personally customized bed 100 include not only the power supply function of the electricity supply unit 104, the communication function of the communications unit 107, and the sensing function of the sensor unit 105 but also an environment adjustment function of the environment-regulating unit 109, an instrument operation adjustment function of the apparatus-regulating unit 110, and an A/V content output adjustment function of the A/Vunit 111.

Here, examples of the personal data of the user are physical state information, private information, health care history information, and A/V content preference information of the user. Examples of the physical state information are biological signals measured by a measurement device of the portable personal terminal 200 and biological signals measured by a measurement device of the personally customized bed 100. That is, the control unit 103 may control a physical and mental relaxation function and a health care function provided by the personally customized bed 100 in accordance with the physical status information of the user, which is obtained from the personal data downloaded by the communications unit 107. Also, if a biological signal measured by the measurement device of the portable personal terminal 200 or a biological signal measured by the measurement device of the personally customized bed 100 indicates an emergency such as a heart attack of the user, the control unit 103 controls the communication function of the communications unit 107 to inform a rescue party (for example, 911) or the physician in charge about the emergency in real time.

In particular, examples of the biological signals are ECG, oxygen saturation (SpO2), electroencephalogram (EEG), blood pressure, pulse rate, breathing rate, and body temperature of the user. However, due to restrictions of volume and weight of the portable personal terminal 200, some of the biological signals may be easily measured by the portable personal terminal 200 while the rest of the biological signals may not. For example, the body temperature of the user may be easily measured if the user holds the portable personal terminal 200 in a hand. In the current embodiment of the present invention, the sensor unit 105 of the personally customized bed 100 may be designed to measure a biological signal which may not be easily measured by the portable personal terminal 200.

Examples of the private information are gender, age, height, weight, and daily schedule of the user. Examples of the health care history information are preference information regarding certain content from among A/V contents, preference information on the environment (for example, temperature, humidity, and illumination level) around the personally customized bed 100, and preference information on a certain massage type by operation of a mechanical frame and the air cells included in the mattress of the personally customized bed 100, which are all represented by a health care history of the user. The health care history information may be generated based on previous health care history information of the user, may be manually set in real time by a health care expert, or may be automatically set by the health monitoring system 800.

With regard to the age from among the private information, the control unit 103 controls the sensing function of the sensor unit 105 to more frequently measure the ECG in proportion to the age. That is, the older the user is, the more frequently the ECG is measured in order to prepare for a sudden heart attack in older aged users. With regard to the height from among the private information, the control unit 103 controls the instrument operation adjustment function of the apparatus-regulating unit 110 to operate the mechanical frame and the air cells included in the mattress of the personally customized bed 100 in accordance with the height of the user. Accordingly, a massage function optimized to the height of the user may be provided.

The electricity supply unit 104 changes a power state of the personally customized bed 100 from the stand-by mode to the active mode, or from the active mode to the stand-by mode, by the control of the control unit 103.

The sensor unit 105 obtains the environment information and the physical state information by sensing the environment around the personally customized bed 100 and a physical state of the user, by the control of the control unit 103. For example, the sensor unit 105 obtains the environment information by sensing humidity, temperature, illumination level, noise level, vibration level, dust amount, and oxygen amount around the personally customized bed 100. Also, the sensor unit 105 obtains the physical state information by sensing the biological signals of the user as described above. In particular, the sensor unit 105 measures a biological signal which may not be easily measured by the portable personal terminal 200, in accordance with selection information of the user, which is received through the user I/F 106. Also, the sensor unit 105 senses a state of the personally customized bed 100 itself by the control of the control unit 103. For example, the sensor unit 105 senses the pressed state of the air cells in the mattress of the personally customized bed 100, or a local clock value of the personally customized bed 100, by the control of the control unit 103.

The user I/F 106 receives selection information indicating whether a biological signal of the user has been measured, and selection information indicating whether to connect the personally customized bed 100 to the health monitoring system 800 located at a remote place, from the user of the portable personal terminal 200. Also, the user I/F 106 receives the direct control information for directly and manually controlling the personally customized bed 100 by the user. Furthermore, if the user-authentication unit 102 determines that the user of the portable personal terminal 200 is not authorized to use the personally customized bed 100, the user I/F 106 may receive new personal data or new user authorization information such as fee pay information.

The communications unit 107 downloads the personal data of the user from the portable personal terminal 200, by the control of the control unit 103. Also, the communications unit 107 may be connected to a network by using a wireless communication function of the portable personal terminal 200, by the control of the control unit 103, may connect the personally customized bed 100 to the health monitoring system 800 located in a remote place such as a node on a WAN, through the connected network or a home network, and may perform consultation with an expert about health care, access an online community through the health monitoring system 800, or receive the remote control information on the personally customized bed 100 from the health monitoring system 800.

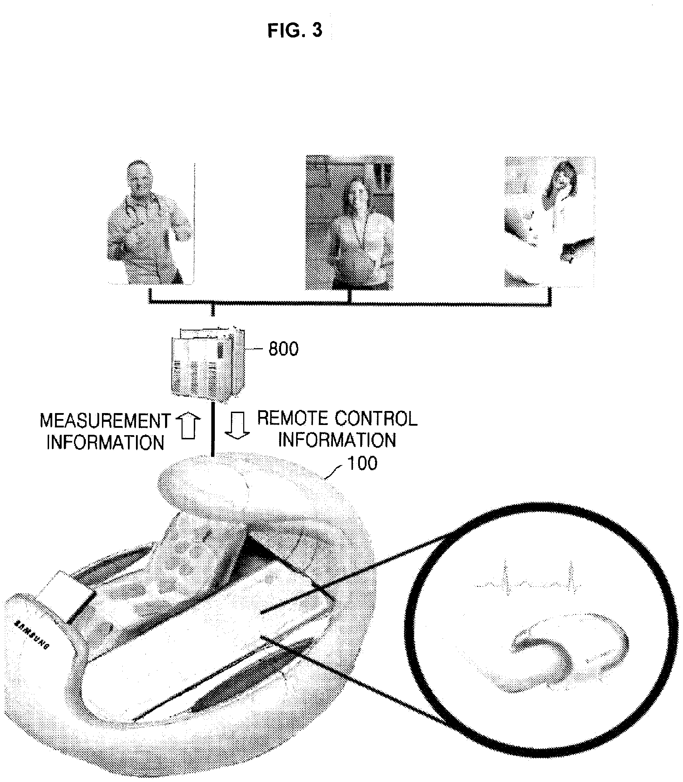

FIG. 3 is a diagram showing an example of a remote medical service provided through the communications unit 107 illustrated in FIG. 2, according to an embodiment of the present invention. FIG. 3 will be described in conjunction with FIG. 2.

Referring to FIG. 3, the communications unit 107 transmits information on biological signals of a user, which are measured by the sensor unit 105, to the health monitoring system 800 through a network. Then, the user may consult with a physician in charge or an expert managing the health monitoring system 800 on line. The physician or the expert inputs remote control information on the personally customized bed 100 to the health monitoring system 800 based on a result of the online consultation and measurement information that is transmitted from the personally customized bed 100 and then the remote control information is transmitted to the personally customized bed 100 through the network. As such, according to the current embodiment of the present invention, the remote medical service may be conveniently and easily provided.

Also, the communications unit 107 may transmit a message stored in the storage unit 108 to a rescue party (for example, 911) or a physician in charge by phone through a PSTN, by the control of the control unit 103. The communications unit 107 may store the downloaded or received information in the storage unit 106 via the control unit 103. Furthermore, the communications unit 107 may function as a library of A/V contents having a large capacity by sequentially and accumulatively storing A/V contents downloaded from the portable personal terminal 200 in the storage unit 108. The storage unit 106 may be implemented as, for example, a hard disk.

Referring back to FIG. 2, the environment-regulating unit 109 adjusts the environment around the personally customized bed 100, by the control of the control unit 103. For example, the environment-regulating unit 109 adjusts at least one of humidity, temperature, illumination level, noise level, vibration level, dust amount, and oxygen amount around the personally customized bed 100. In particular, the environment-regulating unit 109 may include a device having a function for adjusting humidity, temperature, illumination level, noise level, vibration level, dust amount, and oxygen amount around the personally customized bed 100.

The apparatus-regulating unit 110 adjusts instrument operation of the personally customized bed 100, by the control of the control unit 103. In more detail, the apparatus-regulating unit 110 adjusts operation of at least one of the mechanical frame and the air cells included in the mattress of the personally customized bed 100. As illustrated in FIG. 1, the mattress of the personally customized bed 100 includes a mechanical variable frame and a plurality of air cells to physically massage or stretch of the user. In particular, each of the air cells includes a driving instrument for stimulating the body of the user. By using driving instruments, various parts of the body of the user may be separately stimulated. In addition, the apparatus-regulating unit 110 may adjust a releasing amount of an aroma releasing instrument included in the personally customized bed 100, by the control of the control unit 103. Also, the apparatus-regulating unit 110 may move an instrument such as a display panel or a speaker, from an external position to an internal position of the personally customized bed 100, by the control of the control unit 103.

The A/V unit 111 selects A/V content and adjusts an output state of the selected A/V content, by the control of the control unit 103. In more detail, the A/V unit 111 adjusts at least one of an image output of the display panel of the personally customized bed 100 and a speech output of the speaker of the personally customized bed 100, by the control of the control unit 103. For example, the A/V unit 111 may output the daily schedule of the user through the display panel or output psychotherapy music or a fire alarm through the speaker, by the control of the control unit 103.

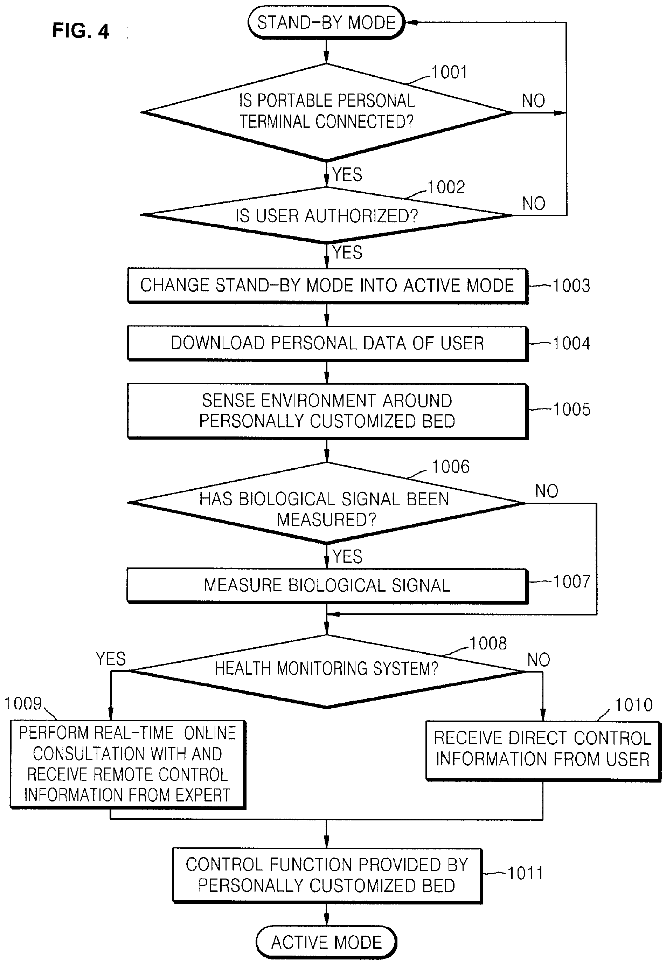

FIG. 4 is a flowchart of a method of implementing a personally customized bed, according to an embodiment of the present invention.

The method according to the current embodiment of the present invention corresponds to a time serial process of the personally customized bed 100 illustrated in FIG. 2. Thus, the method will be described in conjunction with FIG. 2 and repeated descriptions will be omitted.

Referring to FIG. 4, in operation 1001, the terminal I/F 101 detects whether the portable personal terminal 200 is connected to the personally customized bed 100. If it is detected that the portable personal terminal 200 is connected to the personally customized bed 100, the method proceeds to operation 1002. Otherwise, a power state of the personally customized bed 100 is maintained in a stand-by mode.

In operation 1002, the user-authentication unit 102 determines whether a user of the portable personal terminal 200 is authorized to use the personally customized bed 100, by checking a personal identification code of the portable personal terminal 200. If the user-authentication unit 102 determines that the user of the portable personal terminal 200 is authorized to use the personally customized bed 100, the method proceeds to operation 1003. Otherwise, the power state of the personally customized bed 100 is maintained in the stand-by mode.

In operation 1003, the control unit 103 controls a power supply function of the electricity supply unit 104 so that the power state of the personally customized bed 100 is changed from the stand-by mode to an active mode. By the control of the control unit 103, the electricity supply unit 104 changes the power state of the personally customized bed 100 from the stand-by mode to the active mode.

In operation 1004, the control unit 103 controls a communication function of the communications unit 107 to download personal data of the user from the portable personal terminal 200. By the control of the control unit 103, the communications unit 107 downloads the personal data of the user from the portable personal terminal 200.

In operation 1005, the control unit 103 controls a sensing function of the personally customized bed 100 to sense the environment around the personally customized bed 100. By the control of the control unit 103, the sensor unit 105 obtains environment information by sensing the environment around the personally customized bed 100.

In operation 1006, the user I/F 106 receives selection information indicating whether a biological signal of the user has been measured, from the user of the portable personal terminal 200. If the selection information indicates that the biological signal of the user has been measured, the method proceeds to operation 1007. Otherwise, the method proceeds to operation 1008.

In operation 1007, the control unit 103 controls the sensing function of the personally customized bed 100 to measure the biological signal of the user. By the control of the control unit 103, the sensor unit 105 obtains physical state information by sensing the biological signal of the user.

In operation 1008, the user I/F 106 receives selection information indicating whether to connect the personally customized bed 100 to the health monitoring system 800 located in a remote place. If the selection information indicates to connect the personally customized bed 100 to the health monitoring system 800, the method proceeds to operation 1009. Otherwise, the method proceeds to operation 1010.

In operation 1009, the control unit 103 controls the communication function of the personally customized bed 100 to connect the personally customized bed 100 to the health monitoring system 800 through a network. By the control of the control unit 103, the communications unit 107 connects the personally customized bed 100 to the health monitoring system 800 through the network, and performs consultation with an expert about health care or accesses an online community through the health monitoring system 800, or receives remote control information of the expert on the personally customized bed 100 from the health monitoring system 800.

In operation 1010, the user I/F 106 receives direct control information that is input by the user.

In operation 1011, the control unit 103 controls one or more functions provided by the personally customized bed 100, based on at least one of the personal data downloaded in operation 1004, the environment information obtained in operation 1005, the remote control information received in operation 1009, and the direct control information received in operation 1010.

According to the current embodiment of the present invention, a personally optimized electronic bed may be implemented by controlling functions for physical and mental relaxation and for the health care of a user, which are provided by the electronic bed, in accordance with personal data received from a user terminal, for example, information on a physical state of the user, which is measured by the user terminal. For example, environmental factors such as humidity, temperature, and illumination level, a massage function, and A/V content for psychotherapy may be personally optimized and provided to the user. The same effect may be obtained when the current embodiment of the present invention is applied to other types of electronic furniture.

In the above operation 1011, various scenarios are possible for physical and mental relaxation and for the health care of a user. These scenarios may be stored in the storage unit 108 in the form of computer programs. A scenario stored in the storage unit 108 may be implemented by controlling each element of the personally customized bed 100 in accordance with the scenario. In particular, the personally customized bed 100 may be designed so that the user may select one of the scenarios stored in the storage unit 108. A scenario for managing a sound sleep of a user will now be described in detail. Those of ordinary skill in the art may understand that there may exist various scenarios for physical and mental relaxation and for the health care of a user in addition to the scenario to be described below.

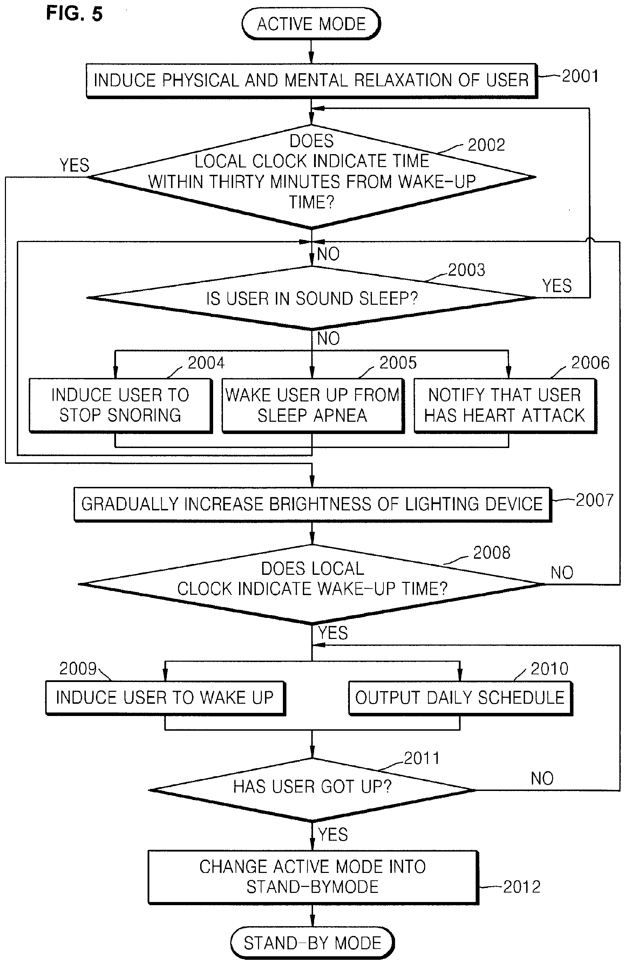

FIG. 5 is a flowchart of a method of managing a sound sleep of a user on the personally customized bed 100 illustrated in FIG. 2, as an example of possible scenarios in operation 1011 illustrated in FIG. 4, according to an embodiment of the present invention. FIG. 5 will be described in conjunction with FIG. 2.

Referring to FIG. 5, in operation 2001, the control unit 103 controls one or more functions provided by the personally customized bed 100 in order to provide physical and mental relaxation to ensure the user has a sound sleep. For example, the control unit 103 may control the environment around the personally customized bed 100 so that brightness of a lighting device attached to the personally customized bed 100 or a lighting device of a room where the personally customized bed 100 is located, is gradually reduced. Here, a degree of the brightness of the lighting device and a variation speed of the brightness of the lighting device are determined by at least one of the personal data downloaded in operation 1004, the environment information obtained in operation 1005, the remote control information received in operation 1009, and the direct control information received in operation 1010, which are described above with reference to FIG. 4.

In operation 2002, the sensor unit 105 determines whether a local clock of the personally customized bed 100 indicates a time within a certain range of time, for example, thirty minutes from wake-up time of the user, by the control of the control unit 103. If the sensor unit 105 determines that the local clock of the personally customized bed 100 indicates a time within the certain range of time from the wake-up time of the user, the method proceeds to operation 2007. Otherwise, the method proceeds to operation 2003. Here, the certain range of time and the wake-up time of the user are determined by at least one of the personal data downloaded in operation 1004, the environment information obtained in operation 1005, the remote control information received in operation 1009, and the direct control information received in operation 1010, which are described above with reference to FIG. 4.

In operation 2003, the sensor unit 105 monitors a sleeping state of the user by measuring biological signals of the user, such as pulse rate and breathing rate, by the control of the control unit 103. If it is determined that the user is in a sound sleep, the method returns to operation 2002. Otherwise, the method proceeds to one of operations 2004 through 2006. In more detail, the method proceeds to operation 2004 if the sensor unit 105 senses a snoring sound from the user, which is above a threshold decibel level, the method proceeds to operation 2005 if the sensor unit 105 senses sleep apnea affecting the user, which lasts above a threshold period of time, and the method proceeds to operation 2006 if the sensor unit 105 senses a heart attack of the user. Here, the threshold decibel level, the threshold period of time, and a reference value of a heart attack are determined by using at least one of the personal data downloaded in operation 1004, the environment information obtained in operation 1005, the remote control information received in operation 1009, and the direct control information received in operation 1010, which are described above with reference to FIG. 4.

In operation 2004, the apparatus-regulating unit 110 vibrates a mattress of the personally customized bed 100 by the control of the control unit 103, or the A/V unit 111 outputs noise through a speaker of the personally customized bed 100 by the control of the control unit 103. As such, the user may be notified so that he or she may stop snoring. Here, an operation type of a mechanical frame and air cells included in the mattress and an output degree of an A/V content are determined by at least one of the personal data downloaded in operation 1004, the environment information obtained in operation 1005, the remote control information received in operation 1009, and the direct control information received in operation 1010, which are described above with reference to FIG. 4.

In operation 2005, the apparatus-regulating unit 110 violently vibrates the mattress of the personally customized bed 100, or the A/V unit 111 outputs a large noise through the speaker of the personally customized bed 100, by the control of the control unit 103. As such, the user may wake up, and thus, the sleep apnea is stopped. Here, the operation type of the mechanical frame and the air cells included in the mattress and the output degree of the A/V content are determined by using at least one of the personal data downloaded in operation 1004, the environment information obtained in operation 1005, the remote control information received in operation 1009, and the direct control information received in operation 1010, which are described above with reference to FIG. 4.

In operation 2006, the communications unit 107 transmits a message indicating that the user has had a heart attack, to a rescue party (for example, 911) or to a physician in charge by phone in real time, by the control of the control unit 103. Here, a phone number of the rescue party or the physician in charge, and an emergency call number are determined by using at least one of the personal data downloaded in operation 1004, the environment information obtained in operation 1005, the remote control information received in operation 1009, and the direct control information received in operation 1010, which are described above with reference to FIG. 4.

In operation 2007, the environment-regulating unit 109 gradually increases brightness of a lighting device attached to the personally customized bed 100 or a lighting device of a room where the personally customized bed 100 is located, by the control of the control unit 103. Here, a degree of the brightness of the lighting device and a variation speed of the brightness of the lighting device are determined by using at least one of the personal data downloaded in operation 1004, the environment information obtained in operation 1005, the remote control information received in operation 1009, and the direct control information received in operation 1010, which are described above with reference to FIG. 4.

In operation 2008, the sensor unit 105 determines whether the local clock of the personally customized bed 100 indicates the wake-up time of the user, by the control of the control unit 103. If the sensor unit 105 determines that the local clock of the personally customized bed 100 indicates the wake-up time of the user, the method proceeds to operation 2009. Otherwise, the method returns to operation 2003.

In operation 2009, the control unit 103 controls the one or more functions provided by the personally customized bed 100 in order to provide physical and mental stimulation, thus inducing the user to wake up. For example, the control unit 103 controls an instrument operation adjustment function of the personally customized bed 100 in such a manner that the body of the user is stretched by operation of the mechanical frame included in the mattress of the personally customized bed 100. Here, an operation type of a health care instrument is determined by using at least one of the personal data downloaded in operation 1004, the environment information obtained in operation 1005, the remote control information received in operation 1009, and the direct control information received in operation 1010, which are described above with reference to FIG. 4.

In operation 2010, the A/V unit 111 outputs a daily schedule of the user through a display panel attached to the personally customized bed 100, by the control of the control unit 103. Here, an output type of an A/V content is determined by using at least one of the personal data downloaded in operation 1004, the environment information obtained in operation 1005, the remote control information received in operation 1009, and the direct control information received in operation 1010, which are described above with reference to FIG. 4.

In operation 2011, the sensor unit 105 determines whether the user has got up by measuring a pressed state of the air cells in the mattress of the personally customized bed 100. If the sensor unit 105 determines that the user has got up, the method proceeds to operation 2012. Otherwise, the method returns to operations 2009 and 2010.

In operation 2012, the electricity supply unit 104 changes a power state of the personally customized bed 100 from an active mode to a stand-by mode, by the control of the control unit 103.

## Claims

1. A method of implementing a personally customized bed (100), the method comprising:
downloading (1004), by the personally customized bed (100), personal data of a user from a user terminal (200) being a portable medical device, wherein the portable medical device is worn on a wrist and measures biological signals; and
controlling (1011), by the personally customized bed (100), one or more functions provided by the personally customized bed (100), based on the personal data, wherein the personal data of the user downloaded from the user terminal (200) includes physical state information based on biological signals measured by a measurement device of the user terminal.

2. The method of claim 1, wherein the controlling comprises controlling the one or more functions provided by the personally customized bed (100), in accordance with a physical state of the user, which is represented by the personal data.

3. The method of claim 2, wherein the physical state comprises at least one of a biological signal of the user, which is measured by the measurement device of the user terminal, and a biological signal of the user, which is measured by a measurement device of the personally customized bed (100).

4. The method of claim 1, wherein the personal data further comprises at least one of private information, health care history information, and audio/video (A/V) content preference information of the user.

5. The method of claim 1, further comprising detecting whether the user terminal is connected to the personally customized bed (100),
wherein the downloading comprises selectively downloading the personal data in accordance with a result of the detecting.

6. The method of claim 1, further comprising receiving remote control information from a health monitoring system which is located in a remote place, through a network, wherein the controlling comprises controlling the one or more functions provided by the personally customized bed (100), based on the remote control information,
wherein the receiving comprises connecting to the network by using a wireless communication function of the user terminal and receiving the remote control information through the network.

7. The method of claim 1, further comprising determining whether the user is authorized to use the personally customized bed (100), by checking a personal identification code from among the personal data,
wherein the controlling comprises controlling the one or more functions provided by the personally customized bed (100), in accordance with a result of the determining.

8. The method of claim 1, wherein the one or more functions comprise an environment adjustment function, an instrument operation adjustment function, and an A/V content output adjustment function of the personally customized bed (100).

9. The method of claim 8, further comprising adjusting at least one of humidity, temperature, illumination level, noise level, vibration level, dust amount, and oxygen amount, around the personally customized bed (100), by controlling the environment adjustment function. wherein the personally customized bed (100) comprises at least one of devices for separately adjusting humidity, temperature, illumination level, noise level, vibration level, dust amount, and oxygen amount, around the personally customized bed (100).

10. The method of claim 8, further comprising adjusting operation of at least one of a mechanical frame and a plurality of air cells included in a mattress of the personally customized bed (100) by controlling the instrument operation adjustment function.

11. The method of claim 8, further comprising adjusting at least one of an image output of a display panel of the personally customized bed (100) and a speech output of a speaker of the personally customized bed (100) by controlling the A/V content output adjustment function.

12. A personally customized bed (100) comprising:
a terminal interface, I/F, (101) adapted for downloading personal data of a user from a user terminal (200) being a portable medical device, wherein the portable medical device is worn on a wrist and measures biological signals; and
a control unit (103) for controlling one or more functions provided by the personally customized bed (100), based on the personal data, wherein the personal data of the user downloaded from the user terminal (200) includes physical state information based on biological signals measured by a measurement device of the user terminal.

13. The personally customized bed (100) of claim 12, wherein the terminal interface I/F comprises a connector into which the user terminal is inserted, and
wherein the personal data is downloaded if the user terminal is connected to the personally customized bed (100) by inserting the user terminal into the connector.

14. A computer-readable recording medium having recorded thereon a computer program for executing a method according to one of claims 1 - 11.

## Patentansprüche

1. Verfahren zum Ausführen eines individuell angepassten Betts (100), wobei das Verfahren umfasst:
Herunterladen (1004) von persönlichen Daten eines Benutzers durch das individuell angepasste Bett (100) von einem Benutzerendgerät (200), das ein tragbares medizinisches Gerät ist, wobei das tragbare medizinische Gerät an einem Handgelenk getragen wird und biologische Signale misst; und
Steuern (1011) von einer oder mehreren durch das individuell angepasste Bett (100) bereitgestellten Funktionen durch das individuell angepasste Bett (100) auf der Basis der persönlichen Daten, wobei die vom Benutzerendgerät (200) heruntergeladenen persönlichen Daten des Benutzers physische Zustandsinformationen auf der Basis von von einer Messvorrichtung des Benutzerendgeräts gemessenen biologischen Signalen umfassen.

2. Verfahren nach Anspruch 1, wobei das Steuern das Steuern der vom individuell angepassten Bett (100) bereitgestellten einen oder mehreren Funktionen gemäß einem physischen Zustand des Benutzers, der durch die persönlichen Daten dargestellt wird, umfasst.

3. Verfahren nach Anspruch 2, wobei der physische Zustand ein biologisches Signal des Benutzers, das von der Messvorrichtung des Benutzerendgeräts gemessen wird, oder/und ein biologisches Signal des Benutzers, das von einer Messvorrichtung des individuell angepassten Betts (100) gemessen wird, umfasst.

4. Verfahren nach Anspruch 1, wobei die persönlichen Daten ferner wenigstens ein Element der Gruppe umfassend private Informationen, Gesundheitsversorgungshistorien-Informationen und Audio/Video-(AV-)Inhaltspräferenzinformationen des Benutzers umfassen.

5. Verfahren nach Anspruch 1, ferner umfassend das Erfassen, ob das Benutzerendgerät mit dem individuell angepassten Bett (100) verbunden ist,
wobei das Herunterladen das selektive Herunterladen der persönlichen Daten gemäß einem Ergebnis des Erfassens umfasst.

6. Verfahren nach Anspruch 1, ferner umfassend das Empfangen einer Fernsteuerungsinformation von einem Gesundheitsüberwachungssystem, das sich an einem externen Ort befindet, über ein Netzwerk,
wobei das Steuern das Steuern der vom individuell angepassten Bett (100) bereitgestellten einen oder mehreren Funktionen auf der Basis der Fernsteuerungsinformation umfasst,
wobei das Empfangen das Verbinden mit dem Netzwerk unter Verwendung einer Drahtloskommunikationsfunktion des Benutzerendgeräts und das Empfangen der Fernsteuerungsinformation über das Netzwerk umfasst.

7. Verfahren nach Anspruch 1, ferner umfassend das Bestimmen, ob der Benutzer zum Verwenden des individuell angepassten Betts (100) autorisiert ist, durch Prüfen eines persönlichen Identifikationscodes in den persönlichen Daten,
wobei das Steuern das Steuern der vom individuell angepassten Bett (100) bereitgestellten einen oder mehreren Funktionen gemäß einem Ergebnis des Bestimmens umfasst.

8. Verfahren nach Anspruch 1, wobei die eine oder mehreren Funktionen eine Umweltanpassungsfunktion, eine Instrumentenbetriebs-Anpassungsfunktion und eine A/V-Inhaltsausgaben-Anpassungsfunktion des individuell angepassten Betts (100) umfassen.

9. Verfahren nach Anspruch 8, ferner umfassend das Anpassen von wenigstens einem Element der Gruppe umfassend Feuchtigkeit, Temperatur, Beleuchtungsniveau, Schallpegel, Schwingungspegel, Staubmenge und Sauerstoffmenge um das individuell angepasste Bett (100) durch Steuern der Umweltanpassungsfunktion,
wobei das individuell angepasste Bett (100) wenigstens eine von Vorrichtungen zum separaten Anpassen von Feuchtigkeit, Temperatur, Beleuchtungsniveau, Schallpegel, Schwingungspegel, Staubmenge und Sauerstoffmenge um das individuell angepasste Bett (100) umfasst.

10. Verfahren nach Anspruch 8, ferner umfassend einen Anpassungsvorgang von einem mechanischen Rahmen oder/und einer Vielzahl von in einer Matratze des individuell angepassten Betts (100) integrierten Luftzellen durch Steuern der Instrumentenbetriebs-Anpassungsfunktion.

11. Verfahren nach Anspruch 8, ferner umfassend das Anpassen einer Bildausgabe eines Anzeigefelds des individuell angepassten Betts (100) oder/und einer Sprachausgabe eines Lautsprechers des individuell angepassten Betts (100) durch Steuern der A/V-Inhaltsausgaben-Anpassungsfunktion.

12. Individuell angepasstes Bett (100), umfassend:
eine zum Herunterladen von persönlichen Daten eines Benutzers von einem Benutzerendgerät (200), das ein tragbares medizinisches Gerät ist, wobei das tragbare medizinische Gerät an einem Handgelenk getragen wird und biologische Signale misst, angepasste Endgerätschnittstelle (101); und
eine Steuereinheit (103) zum Steuern von einer oder mehreren durch das individuell angepasste Bett (100) bereitgestellten Funktionen auf der Basis der persönlichen Daten, wobei die vom Benutzerendgerät (200) heruntergeladenen persönlichen Daten des Benutzers physische Zustandsinformationen auf der Basis von von einer Messvorrichtung des Benutzerendgeräts gemessenen biologischen Signalen umfassen.

13. Individuell angepasstes Bett (100) nach Anspruch 12, wobei die Endgerätschnittstelle einen Steckverbinder umfasst, in den das Benutzerendgerät eingeführt wird, und
wobei die persönlichen Daten heruntergeladen werden, wenn das Benutzerendgerät mit dem individuell angepassten Bett (100) durch Einführen des Benutzerendgeräts in den Steckverbinder verbunden ist.

14. Computerlesbares Aufzeichnungsmedium, auf dem ein Computerprogramm zum Ausführen eines Verfahrens gemäß einem der Ansprüche 1-11 aufgezeichnet ist.

## Revendications

1. Procédé de mise en œuvre d'un lit personnalisé (100), le procédé comprenant:
le téléchargement (1004), par le lit personnalisé (100), de données personnelles d'un utilisateur à partir d'un terminal d'utilisateur (200) qui est un dispositif médical portable, dans lequel le dispositif médical portable est porté au poignet et mesure des signaux biologiques ; et
la commande (1011), par le lit personnalisé (100), d'une ou plusieurs fonctions fournies par le lit personnalisé (100), sur la base des données personnelles, dans lequel les données personnelles de l'utilisateur téléchargées à partir du terminal utilisateur (200) comprennent des informations sur l'état physique basées sur des signaux biologiques mesurés par un dispositif de mesure du terminal utilisateur.

2. Procédé selon la revendication 1, dans lequel le contrôle comprend la commande d'une ou plusieurs fonctions fournies par le lit personnalisé (100), conformément à un état physique de l'utilisateur, qui est représenté par les données personnelles.

3. Procédé selon la revendication 2, dans lequel l'état physique comprend au moins l'un d'un signal biologique de l'utilisateur, qui est mesuré par le dispositif de mesure du terminal de l'utilisateur, et d'un signal biologique de l'utilisateur, qui est mesuré par un dispositif de mesure du lit personnalisé (100).

4. Procédé selon la revendication 1, dans lequel les données personnelles comprennent en outre au moins une des informations privées, des informations sur les antécédents médicaux et des informations sur les préférences de contenu audio/vidéo (A/V) de l'utilisateur.

5. Procédé selon la revendication 1, comprenant en outre la détection du fait que le terminal de l'utilisateur est connecté au lit personnalisé (100),
où le téléchargement comprend le téléchargement sélectif des données personnelles en fonction d'un résultat de la détection.

6. Procédé selon la revendication 1, comprenant en outre la réception d'informations de contrôle à distance d'un système de surveillance de la santé qui est situé dans un lieu éloigné, par l'intermédiaire d'un réseau,
où la commande englobe la commande d'une ou plusieurs fonctions fournies par le lit personnalisé (100), sur la base des informations de la télécommande,
où la réception comprend la connexion au réseau en utilisant une fonction de communication sans fil du terminal de l'utilisateur et la réception des informations de télécommande par le réseau.

7. Procédé selon la revendication 1, comprenant en outre la détermination de l'autorisation de l'utilisateur à utiliser le lit personnalisé (100), en vérifiant un code d'identification personnel parmi les données personnelles,
où la commande englobe la commande d'une ou plusieurs fonctions fournies par le lit personnalisé (100), en fonction d'un résultat de la détermination.

8. Procédé selon la revendication 1, dans lequel la ou les fonctions comprennent une fonction de réglage de l'environnement, une fonction de réglage du fonctionnement de l'instrument et une fonction de réglage de la sortie du contenu audiovisuel du lit personnalisé (100).

9. Procédé selon la revendication 8, comprenant en outre le réglage d'au moins l'un des éléments suivants: humidité, température, niveau d'éclairage, niveau de bruit, niveau de vibration, quantité de poussière et quantité d'oxygène, autour du lit personnalisé (100), en commandant la fonction de réglage de l'environnement, dans lequel le lit personnalisé (100) comprend au moins l'un des dispositifs pour régler séparément l'humidité, la température, le niveau d'éclairage, le niveau de bruit, le niveau de vibration, la quantité de poussière et la quantité d'oxygène, autour du lit personnalisé (100).

10. Procédé selon la revendication 8, comprenant en outre le fonctionnement de réglage d'au moins un parmi un cadre mécanique et une pluralité de cellules d'air incluses dans un matelas du lit personnalisé (100) en commandant la fonction de réglage du fonctionnement de l'instrument.

11. Procédé selon la revendication 8, comprenant en outre le réglage d'au moins une sortie d'image d'un panneau d'affichage du lit personnalisé (100) et d'une sortie vocale d'un locuteur du lit personnalisé (100) en commandant la fonction de réglage de la sortie du contenu A/V

12. Lit personnalisé (100) comprenant:
une interface de terminal, I/F, (101) adaptée pour télécharger des données personnelles d'un utilisateur à partir d'un terminal d'utilisateur (200) qui est un dispositif médical portable, dans lequel le dispositif médical portable est porté au poignet et mesure des signaux biologiques; et
une unité de commande (103) pour commander une ou plusieurs fonctions fournies par le lit personnalisé (100), sur la base des données personnelles, dans lequel les données personnelles de l'utilisateur téléchargées à partir du terminal d'utilisateur (200) comprennent des informations sur l'état physique fondées sur des signaux biologiques mesurés par un dispositif de mesure du terminal d'utilisateur.

13. Lit personnalisé (100) de la revendication 12, dans lequel l'interface du terminal comprend un connecteur dans lequel le terminal d'utilisateur est inséré, et
où les données personnelles sont téléchargées si le terminal utilisateur est connecté au lit personnalisé (100) en insérant le terminal utilisateur dans le connecteur.

14. Support d'enregistrement lisible par ordinateur sur lequel est enregistré un programme informatique pour l'exécution d'un procédé selon l'une des revendications 1 à 11.
